# EUROPEAN PATENT SPECIFICATION

(11) **EP 4 443 157 B1**
(45) Date of publication and mention of the grant of the patent: **23.09.2026**
(21) Application number: 24159481.1
(22) Date of filing: 23.02.2024
(51) Int. Cl.: G01N 33/00, G01N 15/10, A61M 16/08

(54) **AIR QUALITY ANALYSIS DEVICE**
VORRICHTUNG ZUR ANALYSE DER LUFTQUALITÄT
DISPOSITIF D'ANALYSE DE LA QUALITÉ DE L'AIR

(30) Priority: 03.04.2023 US 202318194918
(43) Date of publication of application: 09.10.2024
(73) Proprietor: Honeywell International Inc., Charlotte, NC 28202 (US)
(72) Inventor: AVASTHI, Rahul, Charlotte, 28202 (US)
(74) Representative: Haseltine Lake Kempner LLP

(56) References cited:
- WO-A1-2013/112287
- WO-A1-2021/233957
- WO-A2-2022/043664
- KR-A- 20180 028 336
- US-A1- 2021 369 995

## Description

### TECHNOLOGICAL FIELD

Embodiments of the present disclosure relate generally to an air quality analysis device and a method of performing air quality analysis.

### BACKGROUND

Applicant has identified many technical challenges and difficulties associated with air quality analysis. Through applied effort, ingenuity, and innovation, Applicant has solved problems related to air quality analysis by developing solutions embodied in the present disclosure, which are described in detail below.
US 2021/0369995 A1 discloses methods and systems for determining a quality of a medical gas flow. In one example, a method for a medical gas quality monitoring system includes obtaining measurements of a medical gas via a plurality of sensors, the plurality of sensors including at least one of a humidity sensor, a particulate matter sensor, a carbon dioxide sensor, and a total volatile organic compound sensor, determining a gas quality index of the medical gas based on the obtained measurements, and outputting the determined gas quality index.

### BRIEF SUMMARY

Various embodiments described herein relate to an air quality analysis device and a method of performing air quality analysis.

In accordance with a first aspect, the invention provides a device as defined in claim 1.

In some embodiments, the plurality of sensors further comprises at least one of a particulate matter sensor, a volatile organic compound sensor, a humidity sensor, or a temperature sensor.

In some embodiments, the particulate matter sensor is configured to generate air quality data indicating that there are particles having a diameter of less than 2.5 microns or 10 microns in the air flow

In some embodiments, the plurality of sensors further comprises a first volatile organic compound sensor and a second volatile organic compound sensor.

In some embodiments, the first volatile organic compound sensor is configured to generate air quality data indicating that there is a first volatile organic compound in the air flow and the second volatile organic compound sensor is configured to generate air quality data indicating that there is a second volatile organic compound in the air flow.

In some embodiments, the at least one mold sensor comprises a spectrometer.

The at least one mold sensor is configured to generate air quality data indicating a probability of mold being in the air flow.

In some embodiments, the chamber comprises an outlet port structured to discharge the air flow from the chamber.

In some embodiments, inlet port is structured to receive the air flow into the chamber from a tube associated with a continuous positive airway pressure machine and the outlet port is structured to discharge the air flow from the chamber into a face mask associated with the continuous positive airway pressure machine.

In some embodiments, inlet port is structured to receive the air flow into the chamber from a blower of a continuous positive airway pressure machine and the outlet port is structured to discharge the air flow from the chamber into a tube associated with the continuous positive airway pressure machine.

In some embodiments, the chamber comprises a first portion and a second portion.

In some embodiments, the plurality of sensors is disposed in the first portion.

In accordance with a second aspect, the invention provides a method as defined in claim 10.

In some embodiments, the plurality of sensors further comprises at least one of a particulate matter sensor, a volatile organic compound sensor, a humidity sensor, or a temperature sensor.

In some embodiments, the particulate matter sensor is configured to generate air quality data indicating that there are particles having a diameter of less than 2.5 microns or 10 microns in the air flow.

In some embodiments, the plurality of sensors further comprises a first volatile organic compound sensor and a second volatile organic compound sensor.

In some embodiments, the first volatile organic compound sensor is configured to generate air quality data indicating that there is a first volatile organic compound in the air flow and the second volatile organic compound sensor is configured to generate air quality data indicating that there is a second volatile organic compound in the air flow.

In some embodiments, the at least one mold sensor comprises a spectrometer.

The at least one mold sensor is configured to generate air quality data indicating a probability of mold being in the air flow.

In some embodiments, the chamber comprises an outlet port structured to discharge the air flow from the chamber.

In some embodiments, inlet port is structured to receive the air flow into the chamber from a tube associated with the continuous positive airway pressure machine and the outlet port is structured to discharge the air flow from the chamber into a face mask associated with the continuous positive airway pressure machine.

In some embodiments, inlet port is structured to receive the air flow into the chamber from a blower of the continuous positive airway pressure machine and the outlet port is structured to discharge the air flow from the chamber into a tube associated with the continuous positive airway pressure machine.

In some embodiments, the chamber comprises a first portion and a second portion.

In some embodiments, the plurality of sensors is disposed in the first portion.

For the avoidance of doubt, the scope of the invention is defined by the appended claims.

### BRIEF DESCRIPTION OF THE DRAWINGS

Reference will now be made to the accompanying drawings. The components illustrated in the figures may or may not be present in certain embodiments described herein. Some embodiments may include fewer (or more) components than those shown in the figures in accordance with an example embodiment of the present disclosure.
FIG. 1 illustrates an example air quality analysis device
FIG. 2 illustrates a side view of the example air quality analysis device
FIG. 3 illustrates a cross-sectional view of the example air quality analysis device
FIG. 4 illustrates another side view of the example air quality analysis device
FIG. 5 illustrates another cross-sectional view of the example air quality analysis device
FIG. 6 illustrates an example user interface of a computing device
FIG. 7 illustrates a flowchart of an example method of performing air quality analysis
FIG. 8 illustrates a block diagram of an example computer processing device

### DETAILED DESCRIPTION

Example embodiments will be described more fully hereinafter with reference to the accompanying drawings, in which some, but not all embodiments are shown. Indeed, the invention may be embodied in many different forms and should not be construed as limited to the embodiments set forth herein; rather, these embodiments are provided so that this disclosure will satisfy applicable legal requirements. Like numbers refer to like elements throughout.

### Overview

Example embodiments disclosed herein address technical problems associated with air quality analysis devices and methods of performing air quality analysis, used with a continuous positive airway pressure (CPAP) machine in order to indicate a probably of mold being in the air flow and/or the CPAP machine.

A CPAP machine provides a flow of pressurized air that, in some examples, is also heated and humified, to a user's upper airway while the user is sleeping to ensure that the user's upper airway is not obstructed so the user can obtain enough oxygen while sleeping. In some examples, due to the frequent use of CPAP machines (e.g., every night), the humidification of the air flow, the heating of the air flow, and/or infrequent cleaning by a user of the CPAP machine, CPAP machines often develop mold, resulting in the user being exposed to mold when using the CPAP machine. Accordingly, there is a need for devices and methods that can generate air quality data associated with the air flow provided by CPAP machines to users to ensure that the users do not breath in air flow having mold in it.

### Example Air Quality Analysis Device

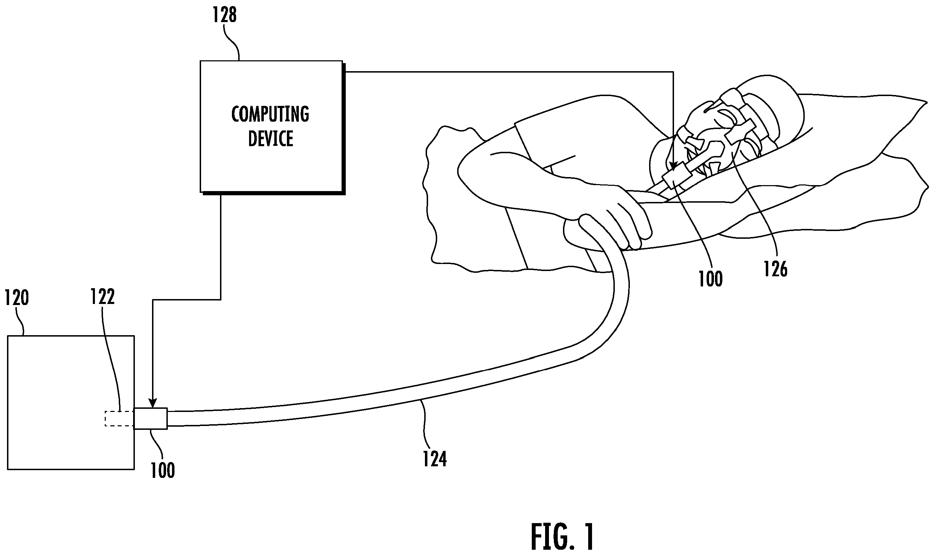

With reference to FIGS. 1-5, an example air quality analysis device 100 is shown that is configured to generate air quality data associated with an air flow. As illustrated in FIG. 1, the air quality analysis device 100 is configured to generate air quality data associated with air flow provided by a continuous positive pressure machine (CPAP) machine 120.

The air quality analysis device 100 includes a housing 102. The housing 102 may be substantially cubical, substantially rectangular, and/or substantially cylindrical. The housing 102 includes a chamber 104. The chamber 104 may be substantially cubical, substantially rectangular, and/or substantially cylindrical. In some embodiments, the chamber 104 may be substantially the same shape as the housing 102. In some embodiments, the chamber 104 may not be substantially the same shape as the housing 102. In some embodiments, the housing 102 and/or the chamber 104 may be comprised of plastic or any other suitable material. In some embodiments, the housing 102 and/or the chamber 104 may be constructed using injection molding and/or 3D printing.

The chamber 104 has an inlet port 106 and an outlet port 108. The inlet port 106 is configured to receive air flow into the chamber 104 and the outlet port 108 is configured to discharge air flow into the chamber 104 (e.g., the air flow moves through the chamber 104 from the inlet port 106 to the outlet port 108). In some embodiments, the inlet port 106 may be structured to receive the air flow into the chamber 104 from a blower 122 of the CPAP machine 120 and the outlet port 108 may be structured to discharge the air to a tube 124 associated with the CPAP machine 120 (e.g., the inlet port 106 may be structured to be attached to the blower 122 of the CPAP machine 120 and the outlet port 108 may be structured to be attached to the tube 124 associated with the CPAP machine 120). Additionally or alternatively, the inlet port 106 may be structured to receive the air flow into the chamber 104 from the tube 124 of the CPAP machine 120 and the outlet port 108 may be structured to discharge the air to a face mask 126 associated with the CPAP machine 120 (e.g., the inlet port 106 may be structured to be attached to the tube 124 of the CPAP machine 120 and the outlet port 108 may be structured to be attached to the face mask 126 associated with the CPAP machine 120).

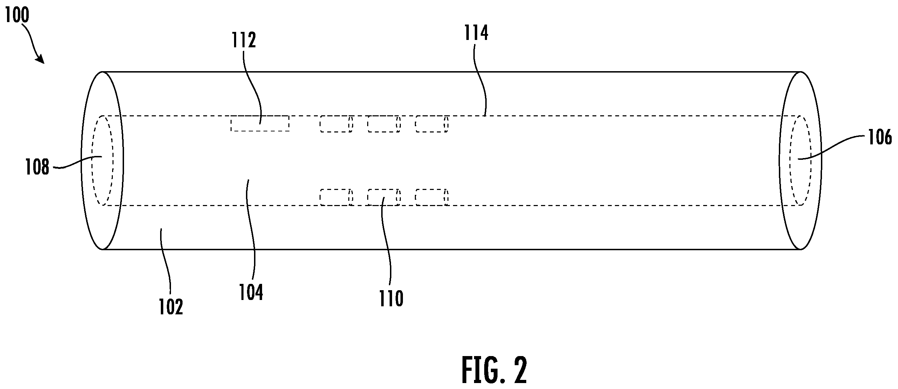

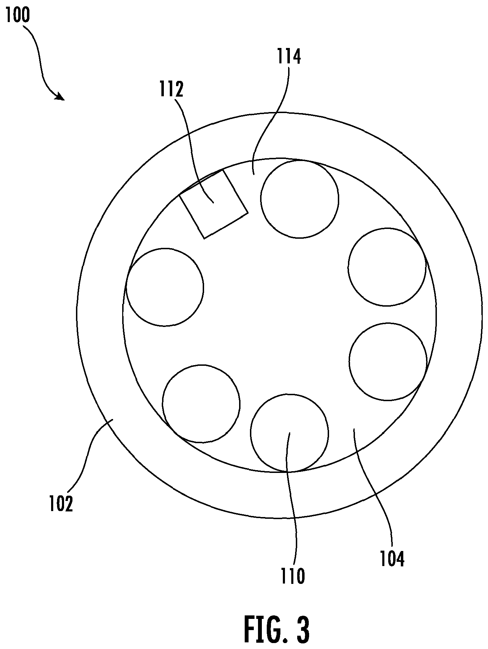

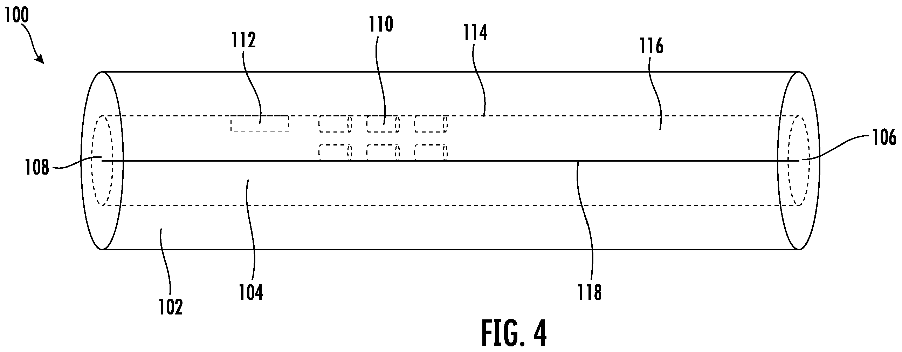

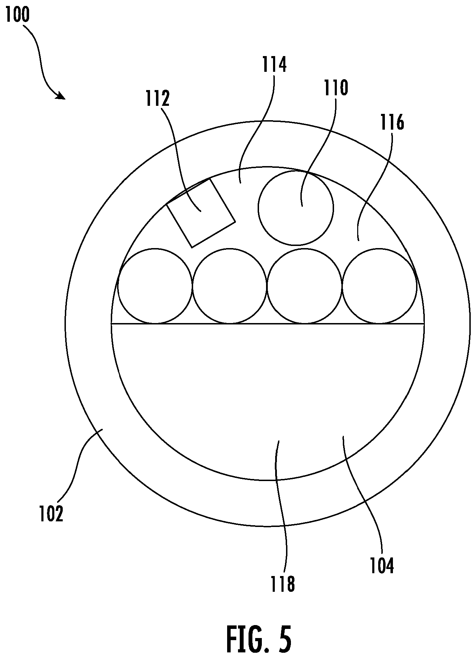

The air quality analysis device 100 includes a plurality of sensors 110 disposed in the chamber 104. The plurality of sensors 110 may be disposed in the chamber 104 in any manner that enables the plurality of sensors 110 to generate air quality data associated with the air flow. Said differently, for example, the plurality of sensors 110 may be disposed in the chamber 104 in any manner that enables each of the plurality of sensors 110 to be able to interact with the air flow such that each of the plurality of sensors 110 can generate air quality data associated with the air flow. For example, such as illustrated in FIGS. 2 and 3, the plurality of sensors 110 may be disposed on an interior surface 114 of the chamber 104. As another example, such as illustrated in FIGS. 4 and 5, the plurality of sensors 110 may be disposed in a first portion 116 of the chamber 104. That is, the plurality of sensors 110 may all be disposed in a first portion 116 of the chamber 104 and none of the plurality of sensors 110 may be disposed in a second portion 118 of the chamber 104.

The sensors 110 are configured to generate air quality data associated with the air flow. In accordance with the invention, the plurality of sensors 110 include at least one mold sensor configured to generate air quality data indicating a probability of mold in the air flow (e.g., is mold present in the air flow). In accordance with the invention, the at least one mold sensor is configured to generate air quality data indicating a probability of mold in the air flow based on a humidity associated with the air flow and a cleaning date associated with the air flow. For example, the mold sensor may be configured to generate air quality data that there is a probability mold in the air flow when the humidity associated with the air flow is a above a humidity threshold (e.g., above 95%) and the cleaning date associated with the air flow indicates that it has been a greater amount of time than a cleaning time threshold (e.g., it has been 6 months since the CPAP Machine 120 has been cleaned and the cleaning time threshold is 3 months). In some embodiments, based on the generated air quality data, the at least one mold sensor may be configured to determine that the probability mold is present in the air flow is low, medium, or high that mold is present in the air flow.

In some embodiments, the plurality of sensors 110 may include at least one particulate matter sensor. In this regard, for example, the plurality of sensors 110 may include at least one particulate matter sensor configured to generate air quality data indicating that there are particles in the air flow (e.g., how many particles in the air flow, concentration of particles in the air flow, size of particles in the air flow, etc.), such as particles having a diameter of less than 2.5 microns and/or 10 microns. In this regard, for example, the plurality of sensors 110 may include a first particulate matter sensor to generate air quality data indicating that there are particles having a diameter of 2.5 microns or less and/or a second particulate matter sensor to generate air quality data indicating that there are particles having a diameter of 10 microns or less. In some embodiments, the plurality of sensors 110 may include a particulate matter sensor that is configured to generate air quality data indicating that there are multiple particles of multiple diameters, such as a particulate matter sensor that can generate air quality data indicating that there are particles having a diameter of 2.5 microns or less and/or particles have a diameter of 10 microns or less.

In some embodiments, the plurality of sensors 110 may include at least one volatile organic compound sensor. In this regard, for example, the plurality of sensors 110 may include at least one volatile organic compound sensor that configured to generate air quality data indicating volatile organic compounds in the air flow, such as one or more of benzene, ethylene glycol, formaldehyde, acetone, methylene chloride, tetrachloroethylene, toluene, xylene, and/or 1,3-butadiene. In some embodiments, the plurality of sensors 110 may include volatile organic compound sensors that are each configured to generate air quality data indicating that there are a specific volatile organic compound is in the air flow. For example, the plurality of sensors 110 may include a first volatile organic compound sensor configured to generate air quality data indicating that there is a first volatile organic compound in the air flow, such as benzene, and a second volatile organic compound sensor configured to generate air quality data indicating that there is a second volatile organic compound, such as acetone. In some embodiments, the plurality of sensors 110 may include a volatile organic compound sensor that is configured to generate air quality data indicating that there are more than one volatile organic compound in the air flow.

In some embodiments, the plurality of sensors 110 may include at least one humidity sensor. In this regard, for example, the plurality of sensors 110 may include at least one humidity sensor that can generate air quality data indicating a humidity associated with the air flow. For example, the at least one humidity sensor may generate air quality data indicating that the air flow is associated with a 90% humidity. In some embodiments, the plurality of sensors 110 may include at least one temperature sensor. In this regard, for example, the plurality of sensors 110 may include at least one temperature sensor that can generate air quality data indicating a temperature associated with the air flow. For example, the at least one temperature sensor may generate air quality data indicating that the air flow has a temperature of 90 degrees.

In some embodiments, the air quality data generated by the plurality of sensors 110 may be obtained and stored in a structured and/or in an unstructured data format. For example, the air quality data may indicate that the probability of mold being in the air flow is low, medium, or high mold is present in the air flow based on the air quality data generated made by the mold sensor. As another example, the air quality data may include a count of the number of times the at least one particulate matter sensor generated air quality data indicating that particles of a particular size are in the air flow (e.g., the at least one particulate matter sensor counted 25 particles with a size of 2.5 microns or less in the air flow). As another example, the air quality data may include an indication of whether or not the at least one volatile organic compound sensor generated air quality data indicating that a specific volatile organic compound is in the air flow (e.g., the at least one volatile organic compound sensor generated air quality data indicating that formaldehyde is in the air flow). As another example, the air quality data may include a temperature and a humidity associated with the air flow as generated by the at least one temperature sensor and/or humidity sensor.

In accordance with the invention, the air quality analysis device 100 includes a printed circuit board (PCB) 112. In some embodiments, the PCB 112 may be disposed proximate the plurality of sensors 110. For example, if the plurality of sensors 110 is disposed on the interior surface 114 of the chamber 104, such as illustrated in FIGS. 2 and 3, the PCB 112 may be disposed on the interior surface 114 of the chamber 104. Additionally or alternatively, if the plurality of sensors is disposed in the first portion 116 of the chamber 104, such as illustrated in FIGS. 4 and 5, the PCB may be disposed in the first portion 116 of the chamber 104.

The PCB 112 is in electrical communication with the plurality of sensors 110 and configured to transmit the air quality data generated by the plurality of sensors 110 to a computing device 128. In some embodiments, the PCB 112 may transmit the air quality data generated by the plurality of sensors 110 to the computing device 128 via Wi-Fi and/or Bluetooth. In some embodiments, the PCB 112 may be configured to transmit the air quality data to the computing device in real-time. For example, each time the at least one volatile organic compound sensor generated air quality data indicating that a volatile organic compound is in the air flow, the PCB 112 may transmit generated air quality data to the computing device 128.

In some embodiments, the PCB 112 may be configured to transmit the air quality data to the computing device 128 on a pre-determined schedule. For example, the PCB 112 may transmit the air quality data to the computing device 128 every 20 seconds. In some embodiments, the PCB 112 may be configured to transmit the air quality data to the computing device 128 at the end of an analysis period. For example, the PCB 112 may be configured to identify when air flow has started flowing through the chamber 104 and when air flow has stopped flowing through the chamber 104. In this regard, when the PCB 112 has identified that the air flow has stopped flowing through the chamber 104, the PCB 112 may transmit the air quality data to the computing device 128. In some embodiments, the PCB 112 may be configured to transmit the air quality data to the computing device 128 upon receiving an instruction to transmit the air quality data from the computing device 128.

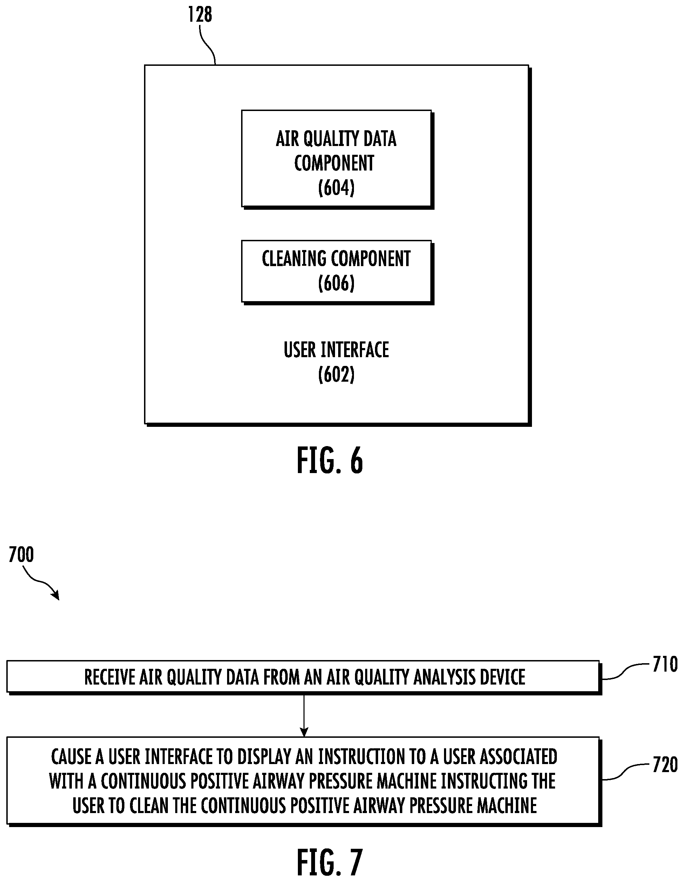

With reference to FIG. 6, in accordance with the invention, the computing device 128 includes a user interface 602. In some embodiments, the user interface 602 may be provided by a mobile application executing on the computing device 128. In some embodiments, the user interface 602 may include an air quality data component 604 configured to indicate air quality data generated by the plurality of sensors 110. For example, the air quality data component 604 may indicate that the probability that there is mold in the air flow is low, medium, or high, the CPAP machine 120, the tube 124, and/or the face mask 126 if the at least one mold sensor determines that the probability that there is mold in the air flow is low, medium, or high. As another example, the air quality data component 604 may indicate a count of the number of times the at least one particulate matter sensor generated air quality data indicating particles of a particular size and the size of the particles in the air flow, the CPAP machine 120, the tube 124, and/or the face mask 126 if the at least one particulate matter sensor determines a count of the number of times the at least one particulate matter sensor generated air quality data indicating particles of a particular size and the size of the particles in the air flow. As another example, the air quality data component 604 may indicate that there is an at least one volatile organic compound sensor in the air flow, the CPAP machine 120, the tube 124, and/or the face mask 126 if the at least one volatile organic compound sensor generated air quality data indicating a specific volatile organic compound is in the air flow (e.g., the at least one volatile organic compound sensor generated air quality data indicating formaldehyde is in the air flow). As another example, the air quality data component 604 may indicate a humidity and/or temperature associated with the air flow the CPAP machine 120, the tube 124, and/or the face mask 126 based on air quality data indicating a humidity of the air flow generated by the at least one humidity sensor and/or air quality data indicating a temperature of the air flow generated by the at least one temperature sensor.

In accordance with the invention, the user interface 602 includes a cleaning component 606 configured to display an instruction indicating that the CPAP machine 120 should be cleaned. The cleaning component 606 displays an instruction indicating that the CPAP machine 120 should be cleaned if the probability that mold is present in the air flow is medium or high. Additionally, the cleaning component 606 may display an instruction indicating that the CPAP machine 120, the tube 124, and/or the face mask 126 should be cleaned if a count of the number of times the at least one particulate matter sensor generated air quality data indicating particles of a particular size in the air flow the CPAP machine 120, the tube 124, and/or the face mask 126 is above a particle threshold. As another example, the cleaning component 606 may display an instruction indicating that the CPAP machine 120, the tube 124, and/or the face mask 126 should be cleaned if the at least one volatile organic compound sensor generated air quality data indicating a specific volatile organic compound (e.g., the at least one volatile organic compound sensor generated air quality data indicating formaldehyde is in the air flow) is in the air flow. As another example, the cleaning component 606 may display an instruction indicating that the CPAP machine 120, the tube 124, and/or the face mask 126 should be cleaned if a temperature and a humidity and/or temperature associated with the air flow the CPAP machine 120, the tube 124, and/or the face mask 126 if a humidity of the air flow is above a humidity threshold and/or a temperature of the air flow is above a temperature threshold.

### Example Method of Performing Air Quality Analysis

Referring now to FIG. 7, a flowchart providing an example method 700 of performing air quality analysis is illustrated. In this regard, FIG. 7 illustrates operations that may be performed by the computing device 128 and/or the air quality analysis device 100.

As shown in block 710, the method 700 of performing air quality analysis may include receiving air quality data from an air quality analysis device. As described above, the air quality analysis device may include a housing. In some embodiments, the housing may include a chamber. In some embodiments, the housing and/or the chamber may be comprised of plastic or any other suitable material. In some embodiments, the housing and/or the chamber may be constructed using injection molding and/or 3D printing.

The chamber has an inlet port and an outlet port. In this regard, the inlet port is configured to receive air flow into the chamber and the outlet port is configured to discharge air flow into the chamber (e.g., the air flow moves through the chamber from the inlet port to the outlet port). In some embodiments, the inlet port may be structured to receive the air flow into the chamber from a blower of the CPAP machine and the outlet port may be structured to discharge the air to a tube associated with the CPAP machine (e.g., the inlet port may be structured to be attached to the blower of the CPAP machine and the outlet port may be structured to be attached to the tube associated with the CPAP machine). Additionally or alternatively, the inlet port may be structured to receive the air flow into the chamber from the tube of the CPAP machine and the outlet port may be structured to discharge the air to a face mask associated with the CPAP machine (e.g., the inlet port may be structured to be attached to the tube of the CPAP machine and the outlet port may be structured to be attached to the face mask associated with the CPAP machine).

As described above, the air quality analysis device includes a plurality of sensors disposed in the chamber. The plurality of sensors may be disposed in the chamber in any manner that enables the plurality of sensors to generate air quality data associated with the air flow. Said differently, for example, the plurality of sensors may be disposed in the chamber in any manner that enables each of the plurality of sensors to be able to interact with the air flow such that each of the plurality of sensors can generate air quality data associated with the air flow. For example, the plurality of sensors may be disposed on an interior surface of the chamber. As another example, the plurality of sensors may be disposed in a first portion of the chamber. That is, the plurality of sensors may all be disposed in a first portion of the chamber and none of the plurality of sensors may be disposed in a second portion of the chamber.

As described above, the plurality of sensors is configured to generate air quality data associated with the air flow. In accordance with the invention, the plurality of sensors is configured to generate air quality data associated with the air flow by generating air quality data indicating that there is a probability of mold in the air flow.

As described above, the air quality data generated by the plurality of sensors may be obtained and stored in a structured and/or in an unstructured data format. In accordance with the invention, the air quality analysis device includes a printed circuit board (PCB). In some embodiments, the PCB may be disposed proximate the plurality of sensors. For example, if the plurality of sensors is disposed on the interior surface of the chamber the PCB may be disposed on the interior surface of the chamber. Additionally or alternatively, if the plurality of sensors is disposed in the first portion of the chamber the PCB may be disposed in the first portion of the chamber.

As described above, the PCB is in electrical communication with the plurality of sensors and be configured to transmit the air quality data generated by the plurality of sensors to a computing device and the computing device is configured to receive the air quality data from the PCB. In some embodiments, the PCB may transmit the air quality data generated by the plurality of sensors to the computing device via Wi-Fi and/or Bluetooth.

As shown in block 720, the method 700 of performing air quality analysis includes causing a user interface to display an instruction to a user associated with a continuous positive airway pressure machine instructing the user to clean the continuous positive airway pressure machine.

As described above, the user interface includes a cleaning component configured to display an instruction indicating that the CPAP machine should be cleaned. For example, the cleaning component may display an instruction indicating that the CPAP machine should be cleaned if the probability that mold is present in the air flow is medium or high. Additionally, the cleaning component may display an instruction indicating that the CPAP machine, the tube, and/or the face mask should be cleaned if the air quality data generated by the at least one particulate matter sensor indicates a count of the particles of a particular size in the air flow the CPAP machine, the tube, and/or the face mask is above a particle threshold. As another example, the cleaning component may display an instruction indicating that the CPAP machine, the tube, and/or the face mask should be cleaned if the at least one volatile organic compound sensor generated air quality data indicating a specific volatile organic compound (e.g., the at least one volatile organic compound sensor generated air quality data indicating formaldehyde is in the air flow) in the air flow. As another example, the cleaning component may display an instruction indicating that the CPAP machine, the tube, and/or the face mask should be cleaned if a temperature and a humidity and/or temperature associated with the air flow the CPAP machine, the tube, and/or the face mask if a humidity of the air flow is above a humidity threshold and/or a temperature of the air flow is above a temperature threshold.

### Example Computer Processing Device

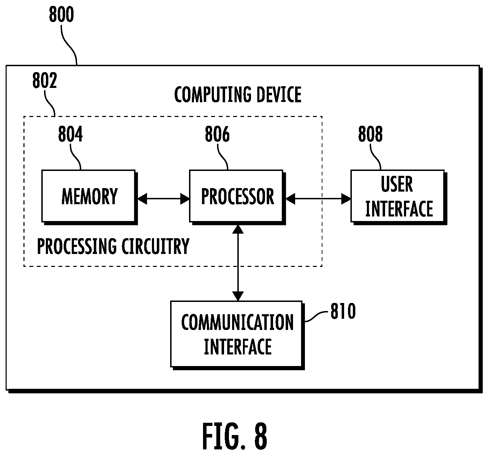

With reference to FIG 8, a block diagram of an example computer processing device 800 is illustrated in accordance with some example embodiments. In some embodiments, the PCB 112, the computing device 128, and/or other devices may be embodied as one or more computer processing devices, such as the computer processing device 800 in FIG. 8.

The computer processing device 800 includes processing circuitry 802 that is configurable to perform actions in accordance with the method of the present invention. In some embodiments, the computer processing device 800 or a portion(s) or component(s) thereof, such as the processing circuitry 802, may be embodied as or comprise a chip or chip set. In other words, the computer processing device 800 or the processing circuitry 802 may comprise one or more physical packages (e.g., chips) including materials, components and/or wires on a structural assembly (e.g., a baseboard). The structural assembly may provide physical strength, conservation of size, and/or limitation of electrical interaction for component circuitry included thereon. The computer processing device 800 or the processing circuitry 802 may therefore, in some cases, be configured to implement an embodiment of the disclosure on a single chip or as a single "system on a chip." As such, in some cases, a chip or chipset may constitute means for performing one or more operations for providing the functionalities described herein.

The processing circuitry 802 includes a processor 806 and memory 804. The processing circuitry 802 is in communication with a user interface 808 and a communication interface 810. As such, the processing circuitry 802 may be embodied as a circuit chip (e.g., an integrated circuit chip) configured (e.g., with hardware, software or a combination of hardware and software) to perform operations described herein.

The processor 806 may be embodied in a number of different ways. For example, the processor 806 may be embodied as various processing means such as one or more of a microprocessor or other processing element, a coprocessor, a controller or various other computing or processing devices including integrated circuits such as, for example, an ASIC (application specific integrated circuit), an FPGA (field programmable gate array), or the like. Although illustrated as a single processor, it will be appreciated that the processor 806 may comprise a plurality of processors. The plurality of processors may be in operative communication with each other and may be collectively configured to perform one or more functionalities of the computer processing device 800 as described herein. In some embodiments, the processor 806 may be configured to execute instructions stored in the memory 804 or otherwise accessible to the processor 806. As such, whether configured by hardware or by a combination of hardware and software, the processor 806 may represent an entity (e.g., physically embodied in circuitry - in the form of processing circuitry 802) capable of performing operations according to embodiments of the present disclosure while configured accordingly. Thus, for example, when the processor 806 is embodied as an ASIC, FPGA or the like, the processor 806 may be specifically configured hardware for conducting the operations described herein. Alternatively, as another example, when the processor 806 is embodied as an executor of software instructions, the instructions may specifically configure the processor 806 to perform one or more operations described herein.

In some embodiments, the memory 804 may include one or more non-transitory memory devices such as, for example, volatile and/or non-volatile memory that may be either fixed or removable. In this regard, the memory 804 may comprise a non-transitory computer-readable storage medium. It will be appreciated that while the memory 804 is illustrated as a single memory, the memory 804 may comprise a plurality of memories. The memory 804 may be configured to store information, data, applications, instructions and/or the like for enabling the computer processing device 800 to carry out various functions in accordance with one or more embodiments. For example, the memory 804 may be configured to buffer input data for processing by the processor 806. Additionally or alternatively, the memory 804 may be configured to store instructions for execution by the processor 806. As yet another alternative, the memory 804 may include one or more databases that may store a variety of files, contents or data sets. Among the contents of the memory 804, applications may be stored for execution by the processor 806 in order to carry out the functionality associated with each respective application. In some cases, the memory 804 may be in communication with one or more of the processor 806, user interface 808, and/or communication interface 810 via a bus(es) for passing information among components of the computer processing device 800.

The user interface 808 is in communication with the processing circuitry 802 to receive an indication of a user input at the user interface 808 and/or to provide an audible, visual, mechanical or other output to the user. As such, the user interface 808 may include, for example, a keyboard, a mouse, a joystick, a display, a touch screen display, a microphone, a speaker, and/or other input/output mechanisms. As such, the user interface 808 provides means for a user to access and interact with the PCB 112 and/or the computing device 128.

The communication interface 810 may include one or more interface mechanisms for enabling communication with other devices and/or networks. In some cases, the communication interface 810 may be any means such as a device or circuitry embodied in either hardware, or a combination of hardware and software that is configured to receive and/or transmit data from/to a network and/or any other device or module in communication with the processing circuitry 802. By way of example, the communication interface 810 may be configured to enable the PCB 112 to communicate with the computing device 128 and/or computing devices. Accordingly, the communication interface 810 may, for example, include an antenna (or multiple antennas) and supporting hardware and/or software for enabling communications with a wireless communication network (e.g., a wireless local area network, cellular network, global positing system network, and/or the like) and/or a communication modem or other hardware/software for supporting communication via cable, digital subscriber line (DSL), universal serial bus (USB), Ethernet or other methods.

Many modifications and other embodiments of the inventions set forth herein will come to mind to one skilled in the art to which these inventions pertain having the benefit of teachings presented in the foregoing descriptions and the associated drawings. Although the figures only show certain components of the apparatus and systems described herein, it is understood that various other components may be used in conjunction with the system. Therefore, it is to be understood that the inventions are not to be limited to the specific embodiments disclosed and that modifications and other embodiments are intended to be included within the scope of the appended claims. Moreover, the steps in the method described above may not necessarily occur in the order depicted in the accompanying diagrams, and in some cases one or more of the steps depicted may occur substantially simultaneously, or additional steps may be involved. Although specific terms are employed herein, they are used in a generic and descriptive sense only and not for purposes of limitation.

For the avoidance of doubt, the scope of the present invention is defined by the appended claims.

Use of broader terms such as "comprises," "includes," and "having" should be understood to provide support for narrower terms such as "consisting of," "consisting essentially of," and "comprised substantially of" Use of the terms "optionally," "may," "might," "possibly," and the like with respect to any element of an embodiment means that the element is not required, or alternatively, the element is required, both alternatives being within the scope of the embodiment(s). Also, references to examples are merely provided for illustrative purposes, and are not intended to be exclusive.

## Claims

1. An air quality analysis device (100), the air quality analysis device comprising:
a housing (102) having a chamber (104) comprising an inlet port (106), wherein the inlet port (106) is structured to receive an air flow into the chamber;
a plurality of sensors (110) disposed within the chamber and configured to generate air quality data associated with the air flow, wherein the plurality of sensors (110) comprises at least one mold sensor,
**characterized in that** the at least one mold sensor is configured to generate the air quality data indicating a probability of mold being in the air flow based at least on a humidity associated with the air flow and a cleaning date associated with the air flow; and further **characterized by**
a printed circuit board (112) in communication with the plurality of sensors and configured to transmit the air quality data to a computing device (128), wherein the computing device comprises a user interface (602) configured to display an instruction to clean a continuous positive airway pressure machine, based on the air quality data.

2. The air quality analysis device (100) of Claim 1, wherein the plurality of sensors (110) further comprises at least one of a particulate matter sensor, a volatile organic compound sensor, a humidity sensor, or a temperature sensor.

3. The air quality analysis device (100) of Claim 2, wherein the particulate matter sensor is configured to the generate air quality data indicating that there are particles having a diameter of less than 2.5 microns or 10 microns in the air flow.

4. The air quality analysis device (100) of Claim 1, wherein the plurality of sensors (110) further comprises a first volatile organic compound sensor and a second volatile organic compound sensor, wherein the first volatile organic compound sensor is configured to generate the air quality data indicating that there is a first volatile organic compound in the air flow and the second volatile organic compound sensor is configured to generate the air quality data indicating that there is a second volatile organic compound in the air flow.

5. The air quality analysis device (100) of Claim 1, wherein the at least one mold sensor comprises a spectrometer.

6. The air quality analysis device (100) of Claim 1, wherein the chamber (104) comprises an outlet port (108) structured to discharge the air flow from the chamber.

7. The air quality analysis device (100) of Claim 6, wherein the inlet port (106) is structured to receive the air flow into the chamber from a tube associated with the continuous positive airway pressure machine and the outlet port (108) is structured to discharge the air flow from the chamber into a face mask associated with the continuous positive airway pressure machine.

8. The air quality analysis device (100) of Claim 6, wherein the inlet port (106) is structured to receive the air flow into the chamber from a blower of the continuous positive airway pressure machine and the outlet port (108) is structured to discharge the air flow from the chamber into a tube associated with the continuous positive airway pressure machine.

9. The air quality analysis device (100) of Claim 1, wherein the chamber (104) comprises a first portion and a second portion, wherein the plurality of sensors (110) is disposed in the first portion.

10. A method (700) of performing an air quality analysis, the method comprising:
receiving (710) air quality data from an air quality analysis device, wherein the air quality analysis device comprises:
a housing having a chamber comprising an inlet port, wherein the inlet port receives an air flow into the chamber;
a plurality of sensors disposed within the chamber and generating air quality data associated with the air flow, wherein the plurality of sensors comprises at least one mold sensor,
**characterized in that** the at least one mold sensor generates the air quality data indicating a probability of mold being in the air flow based at least on a humidity associated with the air flow and a cleaning data associated with the air flow; wherein
a printed circuit board is in communication with the plurality of sensors and
transmits the air quality data to a computing device; and
causing (720) a user interface to display an instruction to a user associated with a continuous positive airway pressure machine instructing the user to clean the continuous positive airway pressure machine.

11. The method (700) of Claim 10, wherein the plurality of sensors further comprises at least one of a particulate matter sensor, a volatile organic compound sensor, a humidity sensor, or a temperature sensor.

12. The method (700) of Claim 10, wherein the at least one mold sensor comprises a spectrometer.

## Patentansprüche

1. Luftqualitätsanalysevorrichtung (100), die Luftqualitätsanalysevorrichtung umfassend:
ein Gehäuse (102), das eine Kammer (104) aufweist, umfassend eine Einlassöffnung (106), wobei die Einlassöffnung (106) dazu strukturiert ist, einen Luftstrom in die Kammer zu empfangen;
eine Vielzahl von Sensoren (110), die innerhalb der Kammer angeordnet und dazu konfiguriert sind, Luftqualitätsdaten zu erzeugen, die dem Luftstrom zugehörig sind, wobei die Vielzahl von Sensoren (110) mindestens einen Schimmelsensor umfasst, **gekennzeichnet dadurch, dass** der mindestens eine Schimmelsensor dazu konfiguriert ist, die Luftqualitätsdaten zu erzeugen, die angeben, dass eine Wahrscheinlichkeit von Schimmel in dem Luftstrom vorliegt, auf Basis mindestens einer Feuchte, die dem Luftstrom zugehörig ist, und ein Reinigungsdatum, das dem Luftstrom zugehörig ist, und ferner **gekennzeichnet durch**
eine Leiterplatte (112), die in Kommunikation mit der Vielzahl von Sensoren und dazu konfiguriert ist, die Luftqualitätsdaten an eine Rechenvorrichtung (128) zu übertragen, wobei die Rechenvorrichtung eine Benutzerschnittstelle (602) umfasst, die dazu konfiguriert ist, auf Basis der Luftqualitätsdaten eine Anweisung anzuzeigen, eine Maschine für kontinuierlichen positiven Atemwegsdruck zu reinigen.

2. Luftqualitätsanalysevorrichtung (100) nach Anspruch 1, wobei die Vielzahl von Sensoren (110) ferner mindestens einen von einem Feinstaubsensor, einem Sensor für flüchtige organische Verbindungen, einem Feuchtesensor oder einem Temperatursensor umfasst.

3. Luftqualitätsanalysevorrichtung (100) nach Anspruch 2, wobei der Feinstaubsensor dazu konfiguriert ist, die Luftqualitätsdaten zu erzeugen, die angeben, dass in dem Luftstrom Partikel vorhanden sind, die einen Durchmesser von weniger als 2,5 Mikrometer oder 10 Mikrometer aufweisen.

4. Luftqualitätsanalysevorrichtung (100) nach Anspruch 1, wobei die Vielzahl von Sensoren (110) ferner einen ersten Sensor für flüchtige organische Verbindungen und einen zweiten Sensor für flüchtige organische Verbindungen umfasst, wobei der erste Sensor für flüchtige organische Verbindungen dazu konfiguriert ist, die Luftqualitätsdaten zu erzeugen, die angeben, dass eine erste flüchtige organische Verbindung in dem Luftstrom vorhanden ist, und der zweite Sensor für flüchtige organische Verbindungen dazu konfiguriert ist, die Luftqualitätsdaten zu erzeugen, die angeben, dass eine zweite flüchtige organische Verbindung in dem Luftstrom vorhanden ist.

5. Luftqualitätsanalysevorrichtung (100) nach Anspruch 1, wobei der mindestens eine Schimmelsensor ein Spektrometer umfasst.

6. Luftqualitätsanalysevorrichtung (100) nach Anspruch 1, wobei die Kammer (104) eine Auslassöffnung (108) umfasst, die dazu strukturiert ist, den Luftstrom aus der Kammer abzuführen.

7. Luftqualitätsanalysevorrichtung (100) nach Anspruch 6, wobei die Einlassöffnung (106) dazu strukturiert ist, den Luftstrom in die Kammer von einem Schlauch, der der Maschine für kontinuierlichen positiven Atemwegsdruck zugehörig ist, zu empfangen, und die Auslassöffnung (108) dazu strukturiert ist, den Luftstrom aus der Kammer in eine Gesichtsmaske, die der Maschine für kontinuierlichen positiven Atemwegsdruck zugehörig ist, abzuführen.

8. Luftqualitätsanalysevorrichtung (100) nach Anspruch 6, wobei die Einlassöffnung (106) dazu strukturiert ist, den Luftstrom in die Kammer von einem Gebläse der Maschine für kontinuierlichen positiven Atemwegsdruck zu empfangen, und die Auslassöffnung (108) dazu strukturiert ist, den Luftstrom aus der Kammer in einen Schlauch, der der Maschine für kontinuierlichen positiven Atemwegsdruck zugehörig ist, abzuführen.

9. Luftqualitätsanalysevorrichtung (100) nach Anspruch 1, wobei die Kammer (104) einen ersten Abschnitt und einen zweiten Abschnitt umfasst, wobei die Vielzahl von Sensoren (110) in dem ersten Abschnitt angeordnet ist.

10. Verfahren (700) zum Durchführen einer Luftqualitätsanalyse, das Verfahren umfassend:
Empfangen (710) von Luftqualitätsdaten von einer Luftqualitätsanalysevorrichtung, wobei die Luftqualitätsanalysevorrichtung Folgendes umfasst:
ein Gehäuse, das eine Kammer aufweist, umfassend eine Einlassöffnung, wobei die Einlassöffnung einen Luftstrom in die Kammer empfängt;
eine Vielzahl von Sensoren, die innerhalb der Kammer angeordnet sind und Luftqualitätsdaten erzeugen, die dem Luftstrom zugehörig sind, wobei die Vielzahl von Sensoren mindestens einen Schimmelsensor umfasst, **gekennzeichnet dadurch, dass** der mindestens eine Schimmelsensor die Luftqualitätsdaten erzeugt, die angeben, dass eine Wahrscheinlichkeit von Schimmel in dem Luftstrom vorliegt, auf Basis mindestens einer Feuchte, die dem Luftstrom zugehörig ist, und Reinigungsdaten, die dem Luftstrom zugehörig sind; wobei
eine Leiterplatte in Kommunikation mit der Vielzahl von Sensoren ist und die Luftqualitätsdaten an eine Rechenvorrichtung überträgt; und
Veranlassen (720), dass eine Benutzerschnittstelle eine Anweisung an einen Benutzer anzeigt, der einer Maschine für kontinuierlichen positiven Atemwegsdruck zugehörig ist, die den Benutzer anweist, die Maschine für kontinuierlichen positiven Atemwegsdruck zu reinigen.

11. Verfahren (700) nach Anspruch 10, wobei die Vielzahl von Sensoren ferner mindestens einen von einem Feinstaubsensor, einem Sensor für flüchtige organische Verbindungen, einem Feuchtesensor oder einem Temperatursensor umfasst.

12. Verfahren (700) nach Anspruch 10, wobei der mindestens eine Schimmelsensor ein Spektrometer umfasst.

## Revendications

1. Dispositif d'analyse de la qualité de l'air (100), le dispositif d'analyse de la qualité de l'air comprenant :
un corps (102) présentant une chambre (104) comprenant un orifice d'entrée (106), dans lequel l'orifice d'entrée (106) est agencé pour recevoir un flux d'air dans la chambre ;
une pluralité de capteurs (110) disposés dans la chambre et configurés pour générer des données de la qualité de l'air associées au flux d'air, dans laquelle la pluralité de capteurs (110) comprend au moins un capteur de moisissures, **caractérisé en ce que** l'au moins un capteur de moisissures est configuré pour générer les données de la qualité de l'air indiquant une probabilité de présence de moisissures dans le flux d'air sur la base d'au moins une humidité associée au flux d'air et d'une date de nettoyage associée au flux d'air ; et **caractérisé en outre en ce que**
une carte de circuit imprimé (112) en communication avec la pluralité de capteurs et configurée pour transmettre les données de la qualité de l'air à un dispositif informatique (128), dans lequel le dispositif informatique comprend une interface utilisateur (602) configurée pour afficher une instruction de nettoyer une machine à pression positive continue, sur la base des données de la qualité de l'air.

2. Dispositif d'analyse de la qualité de l'air (100) selon la revendication 1, dans lequel la pluralité de capteurs (110) comprend au moins un capteur de matières particulaires, un capteur de composés organiques volatils, un capteur d'humidité ou un capteur de température.

3. Dispositif d'analyse de la qualité de l'air (100) selon la revendication 2, dans lequel le capteur de matières particulaires est configuré pour générer les données de la qualité de l'air indiquant qu'il y a des particules présentant un diamètre inférieur à 2,5 microns ou 10 microns dans le flux d'air.

4. Dispositif d'analyse de la qualité de l'air (100) selon la revendication 1, dans lequel la pluralité de capteurs (110) comprend en outre un premier capteur de composés organiques volatils et un deuxième capteur de composés organiques volatils, dans lesquels le premier capteur de composés organiques volatils est configuré pour générer les données de la qualité de l'air indiquant qu'il y a un premier composé organique volatil dans le flux d'air et le deuxième capteur de composés organiques volatils est configuré pour générer les données de la qualité de l'air indiquant qu'il y a un deuxième composé organique volatile dans le flux d'air.

5. Dispositif d'analyse de la qualité de l'air (100) selon la revendication 1, dans lequel l'au moins un capteur de moisissures comprend un spectromètre.

6. Dispositif d'analyse de la qualité de l'air (100) selon la revendication 1, dans lequel la chambre (104) comprend un orifice de sortie (108) agencé pour évacuer le flux d'air à partir de la chambre.

7. Dispositif d'analyse de la qualité de l'air (100) selon la revendication 6, dans lequel l'orifice d'entrée (106) est agencé pour recevoir le flux d'air dans la chambre à partir d'un tube associé à la machine à pression positive continue et l'orifice de sortie (108) est agencé pour évacuer le flux d'air à partir de la chambre dans un masque facial associé à la machine à pression positive continue.

8. Dispositif d'analyse de la qualité de l'air (100) selon la revendication 6, dans lequel l'orifice d'entrée (106) est agencé pour recevoir le flux d'air dans la chambre à partir d'une soufflante de machine à pression positive continue et l'orifice de sortie (108) est agencé pour évacuer le flux d'air à partir de la chambre dans un tube associé à la machine à pression positive continue.

9. Dispositif d'analyse de la qualité de l'air (100) selon la revendication 1, dans lequel la chambre (104) comprend une première partie et une deuxième partie, dans laquelle la pluralité de capteurs (110) est disposée dans la première partie.

10. Procédé (700) de mise en œuvre d'une analyse de la qualité de l'air, le procédé comprenant :
la réception (710) de données de la qualité de l'air à partir d'un dispositif d'analyse de la qualité de l'air, dans lequel le dispositif d'analyse de la qualité de l'air comprend :
un corps présentant une chambre comprenant un orifice d'entrée, dans lequel l'orifice d'entrée reçoit un flux d'air dans la chambre ;
une pluralité de capteurs disposés dans la chambre et générant des données de la qualité de l'air associées au flux d'air, dans laquelle la pluralité de capteurs comprend au moins un capteur de moisissures, **caractérisé en ce que** l'au moins un capteur de moisissures génère les données de la qualité de l'air indiquant une probabilité de présence de moisissures dans le flux d'air sur la base d'au moins une humidité associée au flux d'air et de données de nettoyage associées au flux d'air ; dans lequel
une carte de circuit imprimé est en communication avec la pluralité de capteurs et transmet les données de la qualité de l'air à un dispositif informatique ; et
le fait d'amener (720) une interface utilisateur à afficher à un utilisateur une instruction associée à une machine à pression positive continue donnant instruction à l'utilisateur de nettoyer la machine à pression positive continue.

11. Procédé (700) selon la revendication 10, dans lequel la pluralité de capteurs comprend en outre au moins un capteur de matières particulaires, un capteur de composés organiques volatils, un capteur d'humidité ou un capteur de température.

12. Procédé (700) selon la revendication 10, dans lequel l'au moins un capteur de moisissures comprend un spectromètre.
